(19) Europäisches Patentamt
European Patent Office
Office européen des brevets

(11) EP 1 531 733 B1

(12) # FASCICULE DE BREVET EUROPEEN

(45) Date de publication et mention de la délivrance du brevet:
**12.09.2012 Bulletin 2012/37**

(21) Numéro de dépôt: **03753649.7**

(22) Date de dépôt: **16.07.2003**

(51) Int Cl.:
***A61B 8/08*** *(2006.01)*

(86) Numéro de dépôt international:
**PCT/FR2003/002243**

(87) Numéro de publication internationale:
**WO 2004/016176 (26.02.2004 Gazette 2004/09)**

(54) **DISPOSITIF ET PROCEDE POUR LA MESURE DE L'ELASTICITE D'UN ORGANE HUMAIN OU ANIMAL**

VORRICHTUNG UND VERFAHREN ZUR MESSUNG DER ELASTIZITÄT EINES MENSCHLICHEN ODER TIERISCHEN ORGANS

DEVICE AND METHOD FOR MEASURING ELASTICITY OF A HUMAN OR ANIMAL ORGAN

(84) Etats contractants désignés:
**AT BE BG CH CY CZ DE DK EE ES FI FR GB GR HU IE IT LI LU MC NL PT RO SE SI SK TR**

(30) Priorité: **08.08.2002 FR 0210104**

(43) Date de publication de la demande:
**25.05.2005 Bulletin 2005/21**

(73) Titulaire: **Echosens**
**75005 Paris (FR)**

(72) Inventeurs:
- **SANDRIN, Laurent**
**94240 L'Hay-Les-Roses (FR)**
- **HASQUENOPH, Jean-Michel**
**F-77860 Couilly-Pont-aux-Dames (FR)**

(74) Mandataire: **Lebkiri, Alexandre**
**Cabinet Camus Lebkiri**
**10 rue de la Pépinière**
**75008 Paris (FR)**

(56) Documents cités:
**EP-A- 0 920 833**
**WO-A-00/55616**
**US-A- 5 099 848**
**US-A- 5 882 302**
**US-B1- 6 277 074**

EP 1 531 733 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne un dispositif et un procédé pour la mesure de l'élasticité d'un organe humain ou animal, ou plus généralement tous milieux viscoélastiques présentant un signal ultrasonore après illumination ultrasonore. Elle s'applique en particulier, mais non exclusivement, à la mesure de l'élasticité du foie d'un humain ou d'un animal, l'intérêt de cette mesure étant que cette dernière est corrélée à la quantité de fibrose présente dans le foie.

En effet, les hépatites chroniques qui peuvent être d'origine alcoolique, virale ou autre présentent un effet fibrosant qu'il est important de connaître pour apprécier le meilleur moment pour traiter ces hépatites.

À l'heure actuelle, il n'existe pas sur le marché de dispositifs de mesure de l'élasticité réalisée de manière non invasive, c'est-à-dire par exemple sans prélèvement d'une portion d'organe ou du milieu.

L'art antérieur connaît le brevet US 5882302 qui décrit un transducteur fixé à un moteur. Le moteur permet de déplacer le transducteur de manière à obtenir des images de différentes zones du milieu. Le moteur est donc utilisé pour modifier la zone imagée et absolument pas pour générer un coup basse fréquence. Par ailleurs, le déplacement n'est ici absolument pas parallèle à l'axe du faisceau ultrasonore.

On connaît également le brevet US 6277074 qui décrit un dispositif dans lequel le déplacement du moteur est également parallèle à l'axe ultrasonore. Par ailleurs, ce document ne divulgue pas une acquisition des signaux pendant la compression. En effet, tout comme dans le brevet US 5882302, le moteur est utilisé pour déplacer le transducteur et non pas pour générer un coup basse fréquence.

Le brevet US 5099848 divulgue un dispositif ultrasonore associé à un vibreur utilisé en mode monochromatique de fréquence fixe à 50 Hz. Par ailleurs, dans ce dispositif, le transducteur n'est pas porté par l'actionneur et ne peut donc être utilisé pour générer un coup basse fréquence.

Relativement aux dispositifs les plus récents d'étude et d'analyse de l'élasticité d'un milieu, on connaît déjà la demande internationale de brevet N° WO 0055616 qui décrit un procédé d'imagerie pour observer la propagation d'une onde impulsionnellé de cisaillement basse fréquence simultanément en une multitude de points d'un milieu viscoélastique diffusant. À cet effet, on émet à cadence ultrarapide des ondes ultrasonores de compression qui permettent d'obtenir une succession d'images du milieu, puis on traite en temps différé les images ainsi obtenues par intercorrélation, pour déterminer en chaque point de chaque image les mouvements du milieu lors de la propagation de l'onde de cisaillement. Ce dispositif ne permet pas de localiser la zone dans laquelle est mesurée l'élasticité car il ne fournit pas d'image.

Dans les dispositifs actuels, lorsque le transducteur ultrasonore est utilisé pour engendrer un coup basse fréquence en vibrant mécaniquement, le transducteur est mobile et le référentiel n'est pas fixe. On utilise une technique bien connue de l'homme du métier pour compenser ce déplacement. Cette solution présente plusieurs inconvénients :

- elle nécessite la présence d'un écho ultrasonore provenant d'une zone profonde et immobile du milieu,
- elle est peu précise car le milieu n'étant pas parfaitement immobile, la forme du coup basse fréquence est mal déterminée,
- elle représente un algorithme supplémentaire qui augmente le temps de calcul,
- la surface du milieu présentant une résistance au choc appliqué, la forme réelle de l'impulsion basse fréquence dépend de la pression appliquée par l'opérateur.

Outre les problèmes liés à la compensation du déplacement du vibreur, la pression exercée par l'opérateur est un paramètre qui n'est pas pris en compte alors qu'il perturbe la mesure d'élasticité.

Par ailleurs, l'étude de milieux peu profonds avec un système de type classique en contact direct peut être difficile car la zone focale de certains transducteurs ne permet pas d'obtenir un signal ultrasonore propre, à faible distance du transducteur.

Dans les mesures de déplacements classiques réalisées sur les écoulements sanguins par exemple, l'amplitude des déplacements n'est pas liée à la profondeur dans le milieu mais aux phénomènes observés, par exemple les déplacements liés à l'écoulement du sang sont plus importants au centre de l'artère qu'à ses bords. L'algorithme utilisé pour mesurer les déplacements est donc le même quelle que soit la profondeur. Au contraire en élastographie, l'amplitude des déplacements dépend de la distance à laquelle a été donnée la vibration basse fréquence. Lorsque la vibration est donnée à partir de la surface, l'amplitude des déplacements engendrés par le coup basse fréquence décroît à mesure que l'onde pénètre profondément dans les tissus. L'utilisation d'un algorithme classique n'est pas favorable à la mesure des déplacements sur toute la gamme de profondeur.

L'invention a donc plus particulièrement pour but de remédier aux inconvénients des systèmes de l'art antérieur. Elle propose à cet effet un dispositif pour la mesure de l'élasticité d'un organe humain ou animal, notamment d'un foie, ou plus généralement tous milieux viscoélastiques présentant un signal ultrasonore après illumination ultrasonore, comprenant au moins un palpeur comportant un transducteur ultrasonore, au moins un capteur de position, un actionneur pour le déclenchement dudit dispositif, relié par liaison filaire à une source d'énergie électrique, caractérisé en ce qu'il comprend un actionneur électrodynamique asservi, fixé au transducteur ultrasonore, apte à générer un coup basse

fréquence transitoire présentant une gamme de fréquence comprise entre 1 Hz et 5000 Hz.

On entend par « coup basse fréquence transitoire » une sollicitation mécanique de durée déterminée dont la fréquence est comprise entre 1 Hz et 5000 Hz et dont l'amplitude de crête à crête est comprise entre 10 μm et 20 millimètres, de préférence entre 500 μm et 5 mm. La durée de cette sollicitation est comprise entre 100 μs et 20 secondes, de préférence entre 5 ms et 40 ms (milliseconde).

Grâce à ces particularités, l'invention permet de proposer un dispositif qui permet d'obtenir une vibration, ou sollicitation, basse fréquence parfaitement contrôlée en temps et en amplitude. La connaissance du déplacement exact permet de compenser dans de meilleures conditions et en un minimum de temps le déplacement relatif du vibreur. La forme du coup est mieux contrôlée, ce qui permet d'obtenir des mesures plus fiables et donc d'augmenter la reproductibilité du système. Grâce à l'utilisation de l'actionneur électromagnétique asservi, également dénommé vibreur asservi, le dispositif selon l'invention présente un volume et un poids réduits. Enfin, la présence d'une boucle d'asservissement permet de connaître la pression appliquée par l'opérateur.

Selon une possibilité offerte par l'invention, ce dispositif comprendra un dispositif de protection destiné à assurer la protection du susdit transducteur ultrasonore.

Avantageusement, le dispositif selon l'invention sera commandé par au moins un moyen de commande, par exemple un ordinateur, un micro-ordinateur ou une unité centrale.

De la même manière, le palpeur selon l'invention comprendra une membrane souple et étanche.

Selon un mode d'exécution de l'invention, ce dispositif pour la mesure de l'élasticité d'un organe humain ou animal sera associé à un module d'asservissement et un module d'acquisition ultrasonore aptes à communiquer l'un avec l'autre ; le moyen de commande étant apte à communiquer avec le module d'asservissement et le module d'acquisition ultrasonore.

Selon une possibilité offerte par l'invention, le moyen de commande et l'interface utilisateur seront alimentés en énergie électrique grâce à au moins une batterie.

Avantageusement, ce dispositif comprendra une interface utilisateur, par exemple un écran d'affichage, relié au moyen de commande. Le dispositif sera associé à au moins un échographe ; les images et informations obtenues étant visualisables sur un écran, idéalement celui dudit échographe. Le dispositif pourra être adapté autour d'une barrette échographique. De la même manière, la barrette échographique pourra réaliser elle-même la mesure d'élasticité à condition d'être équipée d'un système vibratoire asservi.

Le dispositif pour la mesure de l'élasticité d'un organe humain ou animal pourra comprendre un milieu intermédiaire élastique transparent aux ultrasons et pour l'onde basse fréquence, tel que par exemple un polymère synthétique de type polyacrylamide.

Avantageusement, au moins l'extrémité du transducteur ultrasonore présentera une forme allongée, par exemple une forme oblongue, rectangulaire, ellipsoïdale, avec une longueur comprise entre 2 et 20 millimètres, de préférence d'environ 11 millimètres, et une largeur comprise entre 1 et 10 millimètres, de préférence d'environ 5 millimètres.

Avantageusement, le transducteur ultrasonore pourra présenter une forme conique ou tronconique présentant un angle compris entre 10 et 80 degrés.

L'invention concerne également un procédé pour le calcul d'une élasticité grâce au susdit dispositif, caractérisé en ce qu'il comprend les étapes suivantes :

- localisation éventuelle par mode image de la zone souhaitée, l'acquisition des signaux ultrasonore, c'est-à-dire pour les lignes écho, pouvant avoir lieu à une cadence d'environ 50 lignes par seconde,
- génération du coup basse fréquence et acquisition des signaux ultrasonores ; l'acquisition pour la mesure de l'élasticité étant réalisée à une cadence élevée entre 100 Hz et 100000 Hz,
- compensation du déplacement relatif du vibreur,
- calcul des vitesses tissulaires, c'est-à-dire les déplacements entre les acquisitions, dans le milieu,
- calcul des vitesses des déformations tissulaires,
- calcul de la vitesse de l'onde élastique,
- calcul de l'élasticité.

Avantageusement, le procédé comprendra une étape préalable de localisation par mode image de la zone souhaitée, l'acquisition des signaux ultrasonore, c'est-à-dire pour les lignes écho, ayant lieu par exemple à une cadence d'environ 50 lignes par seconde. Le résultat obtenu par l'étape de calcul de l'élasticité sera superposé aux lignes écho par exemple sous la forme d'un niveau de couleur différent.

Avantageusement, le procédé comprendra une étape de reconnaissance automatique de l'organe examiné/étudié par le calcul de paramètres tissulaires tels que par exemple le coefficient de rétrodiffusion ultrasonore. La reconnaissance automatique est basée sur le calcul de paramètres tissulaires de l'organe étudié et sur la comparaison de ses paramètres avec les valeurs présentées dans la littérature. À titre d'exemple, le paramètre tissulaire pourra être le coefficient de rétrodiffusion ultrasonore mesuré en temps réel à partir des lignes écho.

Avantageusement, le coup, ou signal, basse fréquence présentera une fréquence comprise entre 1 Hz et 5000 Hz et une durée variant de 1/2f à 20/f.

Des modes d'exécution de l'invention seront décrits ci-après, à titre d'exemple non limitatif, avec référence aux dessins annexés dans lesquels :

- la figure 1 illustre un exemple de dispositif pour la mesure de l'élasticité d'un organe humain ou animal selon l'invention ;
- la figure 2 illustre ledit dispositif muni d'une roulette et d'un moyen de positionnement ultrasonore basse fréquence constitué d'au moins trois récepteurs ultrasonores ;
- la figure 3 illustre un dispositif selon l'invention associé à un échographe
- la figure 4 illustre le dispositif représenté sur la figure 3 associé au palpeur qui est placé sur le côté d'une barrette échographique utilisée pour obtenir l'image du foie et ainsi localisé les zones analysées.
- Les figures 5a à 5d illustrent des mesures d'élasticité superposées à l'image échographique, dans le cas où le dispositif selon l'invention est associé à un échographe, la sonde échographique étant superposée à l'image échographique ;
- la figure 6 illustre un dispositif selon l'invention avec un milieu intermédiaire élastique transparent aux ultrasons
- les figure 7a et 7b illustrent respectivement la forme d'un coup basse fréquence d'amplitude crête à crête 2 millimètres et le spectre fréquentiel du coup basse fréquence dont la fréquence centrale est de 50 Hz et dont la largeur de bande à mi-hauteur s'étend de 18 Hz à 100 Hz, la bande passante atteignant 82 Hz à -6 dB (Décibels).

Selon un exemple choisi pour illustrer l'invention et illustré sur la figure 1, le dispositif selon l'invention comprend un palpeur 1 comportant au moins un transducteur ultrasonore 2, un actionneur électrodynamique 3, un capteur de position 4, une membrane souple et étanche 5, un capuchon de protection 6, un bouton-pression 7 pour le déclenchement du fonctionnement dudit dispositif, les équipements électroniques 8 du capteur de position 4, un câble 9, un afficheur alphanumérique 10.

Le palpeur 1 est commandé par des moyens de commande constitués ici par un micro-ordinateur ou par une unité centrale, non représenté sur les différentes figures, qui peut par exemple être une carte embarquée dans un coffret relié par un câble souple au palpeur 1. Un afficheur, également dénommé interface utilisateur, permet à l'utilisateur ou l'opérateur de lire les informations fournies par le système.

Un module d'asservissement et un module d'acquisition ultrasonore, tous les deux non représentés sur les figures annexées, sont tous deux connectés au palpeur 1. Les deux modules communiquent ensemble ; le module d'acquisition envoyant un signal de synchronisation au moment où une acquisition ultrasonore est déclenchée. La position correspondante est alors enregistrée de manière à pouvoir être communiquée à un algorithme de compensation. L'unité centrale communique avec le module d'acquisition ultrasonore et le module d'asservissement. L'interface utilisateur est constituée d'un écran éventuellement tactile, d'un clavier, éventuellement de curseurs.

L'image du milieu à mesurer peut être affichée sur l'écran de manière à aider l'utilisateur à localiser la zone dans laquelle il veut effectuer la mesure d'élasticité. Le capteur 4 est alors utilisé en mode échographique standard de manière à acquérir typiquement 50 lignes ultrasonores par secondes du milieu. L'enveloppe de ces lignes ultrasonores est affichée à l'écran. Les lignes sont codées en niveau de gris et en échelle logarithmique et placées côte à côte de manière à constituer une image. Le palpeur 1 peut être muni d'un système de positionnement afin de connaître les positions auxquelles sont acquises les lignes et ainsi reconstituer l'image du milieu à mesurer lorsque l'utilisateur, praticien ou opérateur, ballade le palpeur 1 à la surface des tissus humain ou animal.

Nous décrivons ensuite les étapes du procédé selon l'invention qui permettent d'obtenir la mesure d'élasticité ; la succession de ces étapes étant définie selon l'ordre qui suit :

1) localisation éventuelle par mode image de la zone souhaitée, l'acquisition des signaux ultrasonore, c'est-à-dire pour les lignes écho, pouvant avoir lieu à une cadence d'environ 50 lignes par seconde,
2) génération d'un coup basse fréquence et acquisition des signaux ultrasonores ; l'acquisition pour la mesure de l'élasticité étant réalisée à une cadence élevée entre 100 Hz et 100000 Hz,
2) compensation du déplacement relatif du vibreur,
3) calcul des vitesses tissulaires, c'est-à-dire les déplacements entre des acquisitions, dans le milieu,
4) calcul des vitesses des déformations tissulaires,

5) calcul de la vitesse de l'onde élastique
6) calcul de l'élasticité.

Dans le cadre de la génération du coup basse fréquence et de l'acquisition ultrasonore, N acquisitions ultrasonores sont réalisées à une cadence 1/T typiquement comprise entre 100 Hz et 10 000 Hz. Sensiblement au même instant, un signal basse fréquence est transmis au système vibratoire, de préférence juste après le début des acquisitions ultrasonores. Ce signal a une fréquence f, comprise entre 5 Hz et 1000 Hz et une durée variant de 1/2f à 20/f. La vibration basse fréquence entraîne la propagation dans les tissus d'une onde élastique dont la vitesse dépend de l'élasticité du milieu.

L'acquisition des données ultrasonores se fait en émettant avec le transducteur ultrasonore 2 une impulsion ultrasonore qui est réfléchie par les particules contenues dans le milieu. Le signal ultrasonore appelé « speckle » est enregistré par le même transducteur ultrasonore 2 sur une durée pouvant varier entre 1 μs et 10 ms. Cette opération est répétée un nombre N de fois à la cadence 1/T.

Dans tous les modes d'exécution de l'invention, le transducteur est fixé sur le vibreur, ou l'actionneur asservi, ou inversement l'actionneur est fixé sur le transducteur.

Dans l'étape de compensation du déplacement relatif du vibreur, le déplacement des tranches de tissu entre deux acquisitions ultrasonores, d(z,t), est mesuré par rapport à la position du transducteur. Quand ce dernier est immobile, les déplacements mesurés expérimentalement sont égaux aux déplacements absolus. Par contre, quand le transducteur est utilisé pour engendrer l'onde basse fréquence, on doit tenir compte du déplacement du transducteur car les déplacements mesurés expérimentalement ne sont plus égaux aux déplacements absolus. Le déplacement exact du vibreur doit être soustrait des déplacements mesurés pour obtenir les déplacements absolus. Les déplacements mesurés relativement au transducteur s'expriment par :

$$d(z,t) = \delta(z,t) - D(t)$$

où z est la profondeur, D(t) est le déplacement absolu du vibreur et δ(z,t) est le déplacement absolu de la tranche du milieu située à la profondeur z. Le vibreur est placé à la profondeur z = 0.

Par ailleurs comme les déplacements sont dérivés par rapport à la profondeur de manière à obtenir les déformations, le bruit peut devenir important. En effet, la dérivation est très sensible au bruit. Il apparaît donc important de compenser dans de bonnes conditions le déplacement du vibreur. La présence d'un capteur de position 4 permet de mesurer fidèlement et directement D(t). La compensation (ou recalage) des lignes ultrasonores peut par exemple être réalisée dans le domaine de Fourier.

La transformée de Fourier discrète de la ligne ultrasonore numéro m, acquise au temps t=mT est

$$\sum_{n=0}^{N-1} r(m,n)\exp(-j\frac{2\pi nk}{N})$$

où r(m,n) est le signal échantillonné, N est le nombre d'échantillons. Si la ligne ultrasonore a été acquise au temps t=mT, alors la ligne compensée $r_s$ (m, n) s'exprime dans le domaine temporel par

$$r_s(m,n) = \sum_{n=0}^{N-1} R(m,k)\exp(j\frac{2\pi k}{N}(n + \frac{2D(t)}{cT_s}))$$

Dans l'étape de calcul des vitesses tissulaires, les déplacements sont mesurés soit par intercorrélation, par Doppler, soit par autocorrélation et plus généralement par tout autre technique de mesure des déplacements. À titre d'exemple, on peut utiliser l'algorithme d'autocorrélation décrit par Kasai :

$$\delta(z,t=mT) = \frac{V_c}{4\pi f_c}\arg(\sum_{n=p-m}^{n=p+m} \overline{r_s}(m,n)\overline{r_s}^*(m+1,n))$$

où $\bar{r}_s$ est la transformée de Hilbert *de* $r_s$ . $\bar{r}_s$ est le conjugué de $\bar{r}_s$. Avec cet algorithme, on mesure le déplacement δ(z, t) de la tranche de tissu située entre les profondeurs (p-m)Δz et (p+m)Δz entre les temps mT et (m+1)T où T est la période entre deux tirs ultrasonores successifs et Δz le pas d'échantillonnage spatial en profondeur. La vitesse tissulaire v(z,t) s'exprime par

$$v(z,t) = \delta(z,t)/T$$

Dans l'étape de calcul des vitesses de déformation tissulaires, la vitesse de déformation tissulaire est obtenue en dérivant v(z,t) par rapport à la profondeur :

$$\varepsilon(z,t) = \frac{\partial v(z,t)}{\partial z}$$

Dans l'étape de calcul de la vitesse de l'onde élastique, la mesure de la vitesse de l'onde élastique est à titre d'exemple obtenue en calculant la phase φ(z) de l'onde de cisaillement à la fréquence centrale fo de l'onde élastique à chaque profondeur dans le milieu :

$$\varepsilon'(z,f) = FT(\varepsilon(z,t))$$

$$\varphi(z) = \arg(\varepsilon'(z,f_o))$$

$$V_s(z) = \frac{2\pi}{f_o}\left(\frac{d\varphi(z)}{dz}\right)^{-1}$$

Dans l'étape de calcul de l'élasticité, dans les milieux mous comme les tissus biologiques et plus généralement les milieux solides principalement constitués d'eau sous forme liquide, l'élasticité (module d'Young) s'exprime en fonction de la vitesse de cisaillement que nous noterons $V_s$ et de la densité ρ.

$$E = 3\rho V_s^2$$

$$E(z) = 3\rho\left[\frac{2\pi}{f_o}\left(\frac{d\varphi(z)}{dz}\right)^{-1}\right]^2$$

Ainsi, le dispositif pour la mesure de l'élasticité d'un organe humain ou animal fournit soit une valeur moyenne de l'élasticité entre deux profondeurs indiquées par l'utilisateur, soit les variations de l'élasticité en fonction de la profondeur.

Selon une possibilité offerte par l'invention, le palpeur 1 peut comporter plusieurs transducteurs qui peuvent être positionnés de manière arbitraire, par exemple linéairement (type barrette échographique) ou en nid d'abeille. De cette façon, l'élasticité peut être mesurée en différentes zones du milieu à analyser.

En dehors des périodes d'acquisition, le dispositif selon l'invention acquiert des lignes ultrasonores à une cadence typique de 50 lignes par seconde. Ces lignes sont traitées comme en échographie standard de manière à ne conserver que l'enveloppe du signal. Les lignes sont alors affichées sur l'écran du dispositif en niveau de gris et en

échelle logarithmique les unes à la suite des autres et les unes à côté des autres de manière à former une image.

L'image peut être obtenue en déplaçant à vitesse à peu près constante le palpeur 1 à la surface du foie, l'utilisateur disposant alors d'une image déformée de la zone qu'il observe. L'image est déformée car il n'est pas possible pour l'utilisateur de déplacer à vitesse constante le palpeur 1. Cette image lui permet de déterminer le zone dans laquelle la mesure est réalisée. La déformation de l'image est sensiblement réduite en mesurant la position du palpeur 1 à la surface du milieu. Les lignes sont affichées sur l'écran en fonction de l'abscisse du capteur sur le milieu.

Comme illustré sur la figure 2, la position du palpeur 1 à la surface du milieu peut être obtenue à l'aide d'un système de mesure qui peut être de différents types :

- capteur de position du type de ceux utilisés dans les souris pour micro-ordinateur, on peut alors choisir un système utilisant une roulette 11, un système optique comme sur les souris dites « optiques »,
- système de positionnement ultrasonore 12 basse fréquence (typiquement 100 kHz) constitué d'au moins trois récepteurs ultrasonores 13 disposés sur le corps du patient et d'au moins un émetteur 14 placé sur la sonde (la position est obtenue par triangulation)
- ou tout autre système de mesure du déplacement ; le système étant connecté à l'unité centrale.

Le dispositif pour la mesure de l'élasticité d'un organe humain ou animal selon l'invention peut être associé à un échographe 15 standard. Ainsi l'échographe fournit non seulement une information morphologique sur les organes mais également un paramètre quantitatif d'élasticité.

1. L'échographe peut alors présenter en plus des sondes échographiques standards 16, une sonde de type "palpeur" 17, comme illustré sur la figure 5a. Le palpeur 17 peut être adapté autour d'une barrette échographique, non représenté sur les figures, à la manière de certains systèmes de guidage pour biopsie ou d'anciens systèmes de Doppler continu, comme illustré sur la figure 4.

2. Il est également envisageable que la barrette ultrasonore réalise elle-même l'acquisition des signaux ultrasonores utilisés pour l'algorithme d'élastographie.

Avantageusement, le dispositif selon l'invention pourra être portable, qu'il soit branché sur secteur ou alimenté à l'aide de batteries. Un balayage pour la mesure de l'élasticité pourra être opéré manuellement afin d'obtenir une image de l'élasticité. De la même manière, le balayage pourra être réalisé grâce à un moteur pas à pas ou tous autres actionneurs électromagnétiques asservis.

Le système peut éventuellement partager les modules électroniques de l'échographe 15 puisque les échographes 15 standard sont a priori munis d'unités de traitement du signal capables de faire tourner ou calculer les algorithmes nécessaires à la mesure d'élasticité. La barrette peut alors éventuellement engendrer le coup basse fréquence elle-même par un mouvement de vibration qui peut être perpendiculaire à la surface du milieu. L'acquisition peut être réalisée sur la ligne centrale de l'image échographique, comme illustré sur la figure 5a. On peut changer de ligne d'acquisition et reproduire le coup basse fréquence de manière à balayer toute la surface de l'image, comme illustré sur les figures 5b à 5d. Eventuellement on peut réaliser plusieurs lignes en même temps en utilisant des techniques de focalisation ultrasonore évoluées telles que :

• la méthode décrite par Shattuck (cf. « a parrallel processing technique for high speed ultrasound imaging with linear phased arrays », J. Acoust. Soc. Am. 75(4), 1273-1282, 1984),
• une technique de type peigne comme représentée sur les figures 5b à 5d dans laquelle on acquiert simultanément 2, 4 voire 8 lignes. Dans l'exemple de la figure d, les lignes i et i + 64 sont obtenues en même temps.
• une technique de formation de voies ultrarapide en utilisant un algorithme de sommation-retard comme celui décrit dans la demande de brevet N° FR 9903157, d'autres types de « beamforming » comme par exemple la technique dans l'espace des fréquences spatiales.

Il est clair que ce dispositif peut être utilisé conjointement aux techniques d'imagerie ultrarapides décrites dans les documents.cités précédemment de manière à obtenir une image de l'élasticité.

Selon une possibilité offerte par l'invention, le dispositif selon l'invention utilisera un milieu intermédiaire 18 élastique transparent aux ultrasons. Ce milieu 18 peut être par exemple un polymère synthétique de type polyacrylamide. Un matériau adhésif ou une colle pourra être placé entre le milieu intermédiaire 18 et le milieu étudié de manière à obtenir soit une interface glissante, soit une interface liée. Notons que le milieu intermédiaire 18 est innovant car il n'est pas seulement transparent pour les ultrasons mais également pour l'onde basse fréquence. Le milieu intermédiaire 18 est choisi de manière à présenter une élasticité voisine de celle du milieu étudié de manière à adapter l'impédance et ainsi permettre à un maximum d'énergie d'être transmise au milieu étudié. Le milieu intermédiaire 18 peut également

être comprimé pour que son module d'élasticité qui varie de manière non linéaire devienne proche de celui de milieu étudié. Cette dernière proposition est d'ailleurs une technique originale pour mesurer l'élasticité du milieu : elle consiste à modifier l'élasticité du milieu intermédiaire 18 jusqu'à ce qu'un maximum d'énergie soit transmis. L'élasticité atteinte est alors voisine de celle du milieu.

Par ailleurs, le dispositif et procédé de l'invention dispose d'un algorithme ou moyen de calcul des déplacements qui soit adapté en fonction de la profondeur dans le milieu. À faible profondeur, là où l'amplitude des déplacements est grande, l'algorithme compare les lignes successives entre elles. Par contre à forte profondeur, lorsque l'amplitude des déplacements entre lignes successives est faible, la corrélation est effectuée entre la ligne m et la ligne m+Δ avec Δ > 1. En sautant ainsi plusieurs lignes, l'amplitude du déplacement à mesurer grandit et le rapport signal à bruit augmente. L'adaptation de l'algorithme de Kasai donne

$$\delta(z,t=mT)=\frac{V_c}{4\pi f_c}\arg\left(\sum_{n=p-m}^{n=p+m}\overline{r_s}(m,n)\overline{r_s}^{*}(m+\Delta(z),n)\right)$$

où Δ(z) est un nombre entier tel que Δ(z) ≥ 1 qui augmente ave U(z,t)= ondeur.

La connaissance des effets de diffraction associés au vibreur utilisé dans un milieu isotrope voire anisotrope permet de compenser parfaitement les effets de diffraction. On peut également estimer l'atténuation dans le milieu. Dans le cas d'une source de pression basse fréquence en forme de disque, la réponse impulsionnelle de diffraction sur l'axe suit la formule suivante :

$$\begin{cases} \dfrac{2aR^2t}{\rho(z^2+R^2)^{3/2}}, si 0 \le t \langle \dfrac{\sqrt{z^2+R^2}}{V_s} \\ 0, si \dfrac{\sqrt{z^2+R^2}}{V_s} \le t \end{cases}$$

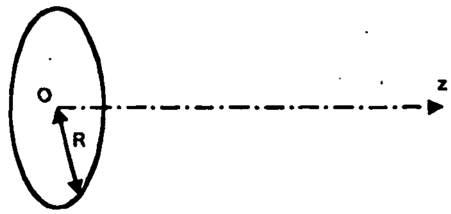


où z est la profondeur sur l'axe du disque, p la densité du milieu, u est le déplacement suivant l'axe de symétrie Oz associé à une contrainte a appliquée suivant Oz, t le temps, R le rayon du disque et $V_s$ la vitesse de cisaillement. On peut introduire l'atténuation α dans cette équation. Cette équation contient à la fois les effets de diffraction et de couplage. Une estimation de $V_s$ voire de α peut être obtenue par un calcul. On peut à titre d'exemple utiliser un calcul itératif d'optimisation qui consiste à minimiser la fonction coût qui est le module de la différence entre les déformations mesurées expérimentalement et celles obtenues avec le modèle théorique.

L'invention est décrite dans ce qui précède à titre d'exemple. Il est entendu que l'homme du métier est à même de réaliser différentes variantes du dispositif et du procédé pour la mesure de l'élasticité d'un organe humain ou animal, en particulier concernant la disposition ou l'agencement des différents éléments constituant ledit dispositif ou l'ordre ainsi que l'importance des étapes dudit procédé, sans pour autant sortir du cadre du brevet.

## Revendications

1. Dispositif pour la mesure de l'élasticité d'un organe humain ou animal, notamment d'un foie, ou plus généralement tous milieux viscoélastiques présentant un signal ultrasonore après illumination ultrasonore, comprenant :

   - au moins un palpeur (1) comportant un transducteur ultrasonore (2),
   - un actionneur pour le déclenchement dudit dispositif, relié par liaison filaire à une source d'énergie électrique,
   - un actionneur électrodynamique (3),

   ledit dispositif étant **caractérisé en ce que** ledit actionneur électrodynamique (3) est asservi et est fixé au transducteur ultrasonore (2), ledit actioneur électrodynamique asservi étant adapté pour générer un coup basse fréquence transitoire présentant une gamme de fréquence comprise entre 1 Hz et 5000 Hz, ledit dispositif comportant en outre :

   - au moins un capteur de position (4) adapté pour mesurer le déplacement absolu de l'actionneur électrodynamique (3) asservi, et
   - des moyens pour compenser le déplacement relatif de l'actionneur électrodynamique (3) asservi.

2. Dispositif selon la revendication 1, **caractérisé en ce qu'**il comprend au moins un moyen de commande, par exemple un ordinateur, un micro-ordinateur ou une unité centrale.

3. Dispositif selon la revendication 1, **caractérisé en ce que** palpeur (1) comprend une membrane souple et étanche (5).

4. Dispositif selon la revendication 1, **caractérisé en ce que** le palpeur (1) comprend un dispositif de protection (6) destiné à protéger le susdit transducteur ultrasonore (2).

5. Dispositif selon la revendication 1, **caractérisé en ce que** le palpeur (1) est associé à un module d'asservissement et un module d'acquisition ultrasonore aptes à communiquer l'un avec l'autre.

6. Dispositif selon les revendications 2 et 5, **caractérisé en ce que** le moyen de commande est apte à communiquer avec le module d'asservissement et le module d'acquisition ultrasonore.

7. Dispositif selon les revendications 1 et 2, **caractérisé en ce qu'**il comprend une interface utilisateur, par exemple un écran d'affichage, relié au moyen de commande.

8. Dispositif selon la revendication 1, **caractérisé en ce que** le palpeur (1) est associé à au moins un échographe (15) ; les images obtenues étant visualisables sur un écran, idéalement celui dudit échographe (15).

9. Dispositif selon la revendication 8, **caractérisé en ce qu'**une barrette échographie est adaptée audit dispositif et qu'elle réalise l'acquisition des signaux ultrasonores.

10. Dispositif selon la revendication 1, **caractérisé en ce qu'**il comprend un système de positionnement ultrasonore (12) constitué d'au moins trois récepteurs disposés sur le corps du patient disposant dudit organe et d'au moins un émetteur (14) placé sur le palpeur (1).

11. Dispositif selon la revendication 1, **caractérisé en ce qu'**il comprend un milieu intermédiaire (18) élastique transparent aux ultrasons et pour l'onde basse fréquence, tel que par exemple un polymère synthétique de type polyacrylamide.

12. Dispositif selon la revendication 11, **caractérisé en ce que** le susdit milieu intermédiaire (18) présente une élasticité voisine de celle du milieu étudié, c'est-à-dire de l'organe humain ou animal à étudier, par exemple en comprimant ce milieu intermédiaire (18) de manière à faire varier son module d'élasticité.

13. Dispositif selon les revendications 2 et 7, **caractérisé en ce que** le moyen de commande et l'interface utilisateur sont alimentés en énergie électrique grâce à au moins une batterie.

14. Dispositif selon la revendication 1, **caractérisé en ce qu'**il comprend un moyen de calcul des déplacements qui soit adapté en fonction de la profondeur dans l'organe humain ou animal.

**15.** Dispositif selon l'une des revendications précédentes, **caractérisé en ce qu'**au moins l'extrémité du transducteur ultrasonore présente une forme allongée, par exemple une forme oblongue, rectangulaire, ellipsoïdale, avec une longueur comprise entre 2 et 20 millimètres, de préférence d'environ 11 millimètres, et une largeur comprise entre 1 et 10 millimètres, de préférence d'environ 5 millimètres.

**16.** Dispositif selon l'une des revendications précédentes, **caractérisé en ce que** le transducteur ultrasonore présente une forme conique ou tronconique présentant un angle compris entre 10 et 80 degrés.

**17.** Procédé pour le calcul d'une élasticité grâce à un dispositif comportant au moins un transducteur ultrasonore (2), un actionneur électrodynamique (3) asservi fixé au transducteur ultrasonore (2), relié par liaison filaire à une source d'énergie électrique, et au moins un capteur de position (4), ledit procédé étant **caractérisé en ce qu'**il comprend les étapes suivantes :

- génération d'un coup basse fréquence transitoire présentant une gamme de fréquence comprise entre 1Hz et 5000Hz et acquisition des signaux ultrasonores,
- compensation du déplacement relatif de l'actionneur électrodynamique asservi en recalant les lignes ultrasonores des signaux ultrasonores,
- calcul des vitesses tissulaires, c'est- à-dire les déplacements entre des acquisitions, dans le milieu,
- calcul des vitesses des déformations tissulaires,
- calcul de la vitesse de l'onde élastique,
- calcul de l'élasticité.

**18.** Procédé pour le calcul d'une élasticité selon la revendication 17, **caractérisé en ce qu'**il comprend une étape préalable de localisation par mode image de la zone souhaitée, l'acquisition des signaux ultrasonore, c'est-à-dire pour les lignes écho, ayant lieu par exemple à une cadence d'environ 50 lignes par seconde.

**19.** Procédé pour le calcul d'une élasticité selon la revendication 18, **caractérisé en ce que** le résultat obtenu par l'étape de calcul de l'élasticité est superposé aux lignes écho par exemple sous la forme d'un niveau de couleur différent.

**20.** Procédé pour le calcul d'une élasticité selon les revendications 17 et 18, **caractérisé en ce que** le coup, ou signal, basse fréquence présente une fréquence comprise entre 1 Hz et 5000 Hz et une durée variant de 1/2f à 20/f.

**21.** Procédé pour le calcul d'une élasticité selon la revendication 17, **caractérisé en ce que** l'étape de calcul de la vitesse tissulaire est réalisée par intercorrélation, par Doppler, par autocorrélation, ou tout autre technique de mesure des déplacements.

**22.** Procédé pour le calcul d'une élasticité selon les revendications 17 et 21, **caractérisé en ce que** le calcul de la vitesse de déformation tissulaire s'effectue en dérivant la vitesse tissulaire par la profondeur.

**23.** Procédé pour le calcul d'une élasticité selon la revendication 17, **caractérisé en ce que** l'acquisition pour la mesure de l'élasticité est réalisée à une cadence élevée entre 100 Hz et 100000 Hz.

**24.** Procédé pour le calcul d'une élasticité selon la revendication 17, **caractérisé en ce qu'**il comprend une étape préalable de balayage de l'organe humain ou animal ; cette étape de balayage pouvant être réalisée de manuellement ou grâce à un moteur pas à pas ou tous autres actionneurs électromagnétiques asservis.

**25.** Procédé pour le calcul d'une élasticité selon l'une quelconque des revendications 17 à 23, **caractérisé en ce qu'**il comprend une étape de reconnaissance automatique de milieu étudié par le calcul de paramètres tissulaires tels que par exemple le coefficient de rétrodiffusion ultrasonore.

## Claims

**1.** A device for measuring the elasticity of a human or animal organ, in particular a liver, or more generally any viscoelastic medium delivering an ultrasonic signal after ultrasonic irradiation, comprising:

- at least one probe (1) comprising an ultrasonic transducer (2);

- an actuator to trigger said device, connected via a wire connection to a source of electrical energy;
- an electrodynamic actuator (3);

said device being **characterized in that** said electrodynamic actuator (3) is controlled and is attached to the ultrasonic transducer (2), said controlled electrodynamic actuator being adapted to generate a transient low frequency pulse having a frequency range in the range 1 Hz to 5000 Hz, said device further comprising:

- at least one position sensor (4) adapted to measure the absolute displacement of the controlled electrodynamic actuator (3); and
- means for compensating for the relative displacement of the controlled electrodynamic actuator (3).

**2.** The device as claimed in claim 1, **characterized in that** it comprises at least one control means, for example a computer, a microcomputer or a central processing unit.

**3.** The device as claimed in claim 1, **characterized in that** the probe (1) comprises a flexible, impervious membrane (5).

**4.** The device as claimed in claim 1, **characterized in that** the probe (1) comprises a protective device (6) for protecting said ultrasonic transducer (2).

**5.** The device as claimed in claim 1, **characterized in that** the probe (1) is associated with a control module and with an ultrasonic acquisition module which are capable of communicating with each other.

**6.** The device as claimed in claims 2 and 5, **characterized in that** the control means is capable of communicating with the control module and with the ultrasonic acquisition module.

**7.** The device as claimed in claims 1 and 2, **characterized in that** it comprises a user interface, for example a display screen, connected to the control means.

**8.** The device as claimed in claim 1, **characterized in that** the probe (1) is associated with at least one echograph (15), with the images obtained being capable of being viewed on a screen, ideally that of said echograph (15).

**9.** The device as claimed in claim 8, **characterized in that** an echographic bar is adapted to said device and it carries out the acquisition of the ultrasonic signals.

**10.** The device as claimed in claim 1, **characterized in that** it comprises an ultrasonic positioning system (12) constituted by at least three receivers disposed on the body of the patient with said organ and at least one transmitter (14) placed on the probe (1).

**11.** The device as claimed in claim 1, **characterized in that** it comprises an elastic intermediate medium (18) which is transparent to ultrasonic and to the low frequency wave, such as a synthetic polyacrylamide type polymer, for example.

**12.** The device as claimed in claim 11, **characterized in that** the elasticity of said intermediate medium (18) is close to that of the medium being studied, i.e. the human or animal organ to be studied, for example by compressing said intermediate medium (18) so as to vary its modulus of elasticity.

**13.** The device as claimed in claims 2 and 7, **characterized in that** the control means and the user interface are supplied with electrical energy by means of at least one battery.

**14.** The device as claimed in claim 1, **characterized in that** it comprises a means for computing displacements which is adapted to operate as a function of the depth in the human or animal organ.

**15.** The device as claimed in one of the preceding claims, **characterized in that** at least one end of the ultrasonic transducer has an elongate shape, for example an oblong, rectangular or ellipsoidal shape, with a length in the range 2 to 20 millimetres, preferably approximately 11 millimetres, and a width in the range 1 to 10 millimetres, preferably approximately 5 millimetres.

**16.** The device as claimed in one of the preceding claims, **characterized in that** the ultrasonic transducer has a conical

or truncated shape with an angle in the range 10 to 80 degrees.

**17.** A method for computing an elasticity by means of a device comprising at least one ultrasonic transducer (2), a controlled electrodynamic actuator (3) attached to the ultrasonic transducer (2), connected via a wire connection to a source of electrical energy, and at least one position sensor (4), said method being **characterized in that** it comprises the following steps:

- generating a transient low frequency pulse having a frequency range in the range 1 Hz to 5000 Hz and acquiring ultrasonic signals;
- compensating for the relative displacement of the controlled electrodynamic actuator by recalibrating the ultrasonic lines of the ultrasonic signals;
- computing the tissue velocities, i.e. the displacements between acquisitions, in the medium;
- computing the velocities of tissue deformations;
- computing the velocity of the elastic wave;
- computing the elasticity.

**18.** The method for computing an elasticity as claimed in claim 17, **characterized in that** it comprises a prior step of localizing the desired zone in image mode, with acquisition of the ultrasonic signals, i.e. the echo lines, taking place at a cadence of approximately 50 lines per second, for example.

**19.** The method for computing an elasticity as claimed in claim 18, **characterized in that** the result obtained by the elasticity computation step is superimposed on the echo lines, for example in the form of a different colour level.

**20.** The method for computing an elasticity as claimed in claims 17 and 18, **characterized in that** the frequency of the pulse or low frequency signal is in the range 1 Hz to 5000 Hz and the duration is in the range 1/2f to 1/20f.

**21.** The method for computing an elasticity as claimed in claim 17, **characterized in that** the step for computing the tissue velocity is carried out by intercorrelation, by the Doppler effect, by autocorrelation, or by any other technique for measuring displacements.

**22.** The method for computing an elasticity as claimed in claims 17 and 21, **characterized in that** the tissue deformation velocity is computed by taking the derivative of the tissue velocity with respect to depth.

**23.** The method for computing an elasticity as claimed in claim 17, **characterized in that** the elasticity measurement is acquired at a high cadence in the range 100 Hz to 100000 Hz.

**24.** The method for computing an elasticity as claimed in claim 17, **characterized in that** it comprises a prior step of scanning the human or animal organ, this scanning step possibly being carried out manually or by means of a step motor or any other controlled electromagnetic actuators.

**25.** The method for computing an elasticity as claimed in claims 17 to 23, **characterized in that** it comprises a step for automatic recognition of the medium being studied by computing tissue parameters such as, for example, the coefficient of ultrasonic back-scatter.

## Patentansprüche

**1.** Vorrichtung zum Messen der Elastizität eines menschlichen oder tierischen Organs, insbesondere einer Leber, oder allgemeiner aller viskoelastischen Medien, die nach Ultraschallbeleuchtung ein Ultraschallsignal aufweisen, umfassend:

- mindestens einen Fühler (1), umfassend einen Ultraschallwandler (2),
- ein Stellglied zum Auslösen der Vorrichtung, das durch eine Kabelverbindung mit einer Stromquelle verbunden ist,
- ein elektrodynamisches Stellglied (3),

wobei die Vorrichtung **dadurch gekennzeichnet ist, dass** das elektrodynamische Stellglied (3) vom Ultraschallwandler (2) geregelt wird und an diesem befestigt ist, wobei das geregelte elektrodynamische Stellglied angepasst

ist, um einen vorübergehenden Niederfrequenzimpuls zu erzeugen, der einen Frequenzbereich zwischen 1 Hz und 5.000 Hz aufweist, wobei die Vorrichtung ferner umfasst:

- mindestens einen Positionssensor (4), der angepasst ist, um die absolute Verschiebung des geregelten elektrodynamischen Stellglieds (3) zu messen, und
- Mittel zum Ausgleichen der relativen Verschiebung des gesteuerten elektrodynamischen Stellglieds (3).

2. Verfahren nach Anspruch 1, **dadurch gekennzeichnet, dass** es mindestens ein Steuerungsmittel, zum Beispiel einen Computer, einen Mikrocomputer oder eine Zentraleinheit umfasst.

3. Verfahren nach Anspruch 1, **dadurch gekennzeichnet, dass** der Fühler (1) eine flexible und dichte Membran (5) umfasst.

4. Verfahren nach Anspruch 1, **dadurch gekennzeichnet, dass** der Fühler (1) eine Schutzvorrichtung (6) umfasst, die dazu bestimmt ist, den oben genannten Ultraschallwandler (2) zu schützen.

5. Vorrichtung nach Anspruch 1, **dadurch gekennzeichnet, dass** der Fühler (1) mit einem Regelungsmodul und einem Ultraschallerfassungsmodul assoziiert ist, die fähig sind, miteinander zu kommunizieren.

6. Vorrichtung nach den Ansprüchen 2 und 5, **dadurch gekennzeichnet, dass** das Steuerungsmittel fähig ist, mit dem Regelungsmodul und dem Ultraschallerfassungsmodul zu kommunizieren.

7. Vorrichtung nach den Ansprüchen 1 und 2, **dadurch gekennzeichnet, dass** sie eine Nutzerschnittstelle, zum Beispiel einen Anzeigebildschirm, umfasst, der mit dem Steuerungsmittel verbunden ist.

8. Vorrichtung nach Anspruch 1, **dadurch gekennzeichnet, dass** der Fühler (1) mit mindestens einem Echographen (15) assoziiert ist; wobei die erhaltenen Bilder auf einem Bildschirm, idealerweise dem des Echographen (15), sichtbar gemacht werden können.

9. Vorrichtung nach Anspruch 8, **dadurch gekennzeichnet, dass** ein Echographiestab an die Vorrichtung angepasst ist und dass er die Erfassung der Ultraschallsignale ausführt.

10. Vorrichtung nach Anspruch 1, **dadurch gekennzeichnet, dass** sie ein Ultraschallpositionierungssystem (12), das aus mindestens drei Empfängern besteht, die auf dem Körper des Patienten, der mit dem Organ versehen ist, angeordnet sind, und mindestens einen Sender (14), der auf dem Fühler (1) angebracht ist, umfasst.

11. Vorrichtung nach Anspruch 1, **dadurch gekennzeichnet, dass** sie ein elastisches Zwischenmedium (18) umfasst, das für Ultraschallwellen und für die Niederfrequenzwelle durchlässig ist, wie zum Beispiel ein synthetisches Polymer vom Typ Polyacrylamid.

12. Vorrichtung nach Anspruch 11, **dadurch gekennzeichnet, dass** das oben genannte Zwischenmedium (18) eine Elastizität ähnlich derjenigen des untersuchten Mediums, das heißt, dem zu untersuchenden menschlichen oder tierischen Organ, aufweist, zum Beispiel, indem dieses Zwischenmedium (18) komprimiert wird, um sein Elastizitätsmodul variieren zu lassen.

13. Vorrichtung nach den Ansprüchen 2 und 7, **dadurch gekennzeichnet, dass** das Steuerungsmittel und die Nutzerschnittstelle mithilfe mindestens einer Batterie mit Strom versorgt werden.

14. Vorrichtung nach Anspruch 1, **dadurch gekennzeichnet, dass** sie ein Mittel zum Berechnen der Verschiebungen umfasst, das in Abhängigkeit von der Tiefe in dem menschlichen oder tierischen Organ angepasst wird.

15. Vorrichtung nach einem der vorhergehenden Ansprüche, **dadurch gekennzeichnet, dass** mindestens das Ende des Ultraschallwandlers eine gestreckte Form, zum Beispiel eine längliche, rechteckige, elliptische Form, mit einer Länge zwischen 2 und 20 Millimetern, bevorzugt von etwa 11 Millimetern, und einer Breite zwischen 1 und 10 Millimetern, bevorzugt von etwa 5 Millimetern, aufweist.

16. Vorrichtung nach einem der vorhergehenden Ansprüche, **dadurch gekennzeichnet, dass** der Ultraschallwandler eine konische oder kegelstumpfförmige Form aufweist, die einen Winkel zwischen 10 und 80 Grad aufweist.

**17.** Verfahren zum Berechnen einer Elastizität mithilfe einer Vorrichtung, umfassend mindestens einen Ultraschallwandler (2), ein geregeltes elektrodynamisches Stellglied (3), das am Ultraschallwandler (2) befestigt ist, das über eine Kabelverbindung mit einer Stromquelle verbunden ist, und mindestens einen Positionssensor (4), wobei das Verfahren **dadurch gekennzeichnet ist, dass** es die folgenden Schritte umfasst:

- Erzeugen eines vorübergehenden Niederfrequenzimpulses, der einen Frequenzbereich zwischen 1 Hz und 5.000 Hz aufweist, und Erfassen der Ultraschallsignale,
- Ausgleichen der relativen Verschiebung des gesteuerten elektrodynamischen Stellglieds, indem die Ultraschalllinien der Ultraschallsignale neu ausgerichtet werden,
- Berechnen der Gewebegeschwindigkeiten, das heißt, der Verschiebungen zwischen Erfassungen, in dem Medium,
- Berechnen der Geschwindigkeiten der Gewebeverformungen,
- Berechnen der Geschwindigkeit der elastischen Welle,
- Berechnen der Elastizität.

**18.** Verfahren zum Berechnen einer Elastizität nach Anspruch 17, **dadurch gekennzeichnet, dass** es einen vorausgehenden Schritt zur Lokalisierung der gewünschten Zone durch Abbildungsmodus, die Erfassung der Ultraschallsignale, das heißt, für die Echolinien, die zum Beispiel bei einem Takt von etwa 50 Linien pro Sekunde auftreten, umfasst.

**19.** Verfahren zum Berechnen einer Elastizität nach Anspruch 18, **dadurch gekennzeichnet, dass** das erhaltene Ergebnis, das durch den Schritt zum Berechnen der Elastizität erhalten wird, über die Echolinien gelegt wird, zum Beispiel in Form einer unterschiedlichen Farbintensität.

**20.** Verfahren zum Berechnen einer Elastizität nach den Ansprüchen 17 und 18, **dadurch gekennzeichnet, dass** der Niederfrequenzimpuls oder das Niederfrequenzsignal eine Frequenz zwischen 1 Hz und 5.000 Hz und einer Dauer aufweist, die von 1/2f bis 20/f variiert.

**21.** Verfahren zum Berechnen einer Elastizität nach Anspruch 17, **dadurch gekennzeichnet, dass** der Schritt zum Berechnen der Gewebegeschwindigkeit durch Interkorrelation, durch Doppler, durch Autokorrelation oder jede andere Technik zum Messen der Verschiebungen ausgeführt wird.

**22.** Verfahren zum Berechnen einer Elastizität nach den Ansprüchen 17 und 21, **dadurch gekennzeichnet, dass** das Berechnen der Geschwindigkeit der Gewebeverformung erfolgt, indem die Gewebegeschwindigkeit mittels der Tiefe abgeleitet wird.

**23.** Verfahren zum Berechnen einer Elastizität nach Anspruch 17, **dadurch gekennzeichnet, dass** das Erfassen zum Messen der Elastizität mit einem hohen Takt zwischen 100 Hz und 100.000 Hz ausgeführt wird.

**24.** Verfahren zum Berechnen einer Elastizität nach Anspruch 17, **dadurch gekennzeichnet, dass** es einen vorhergehenden Schritt zum Abtasten des menschlichen oder tierischen Organs umfasst; wobei dieser Schritt zum Abtasten manuell oder mithilfe eines Schrittmotors oder allen anderen geregelten elektromagnetischen Stellgliedern ausgeführt werden kann.

**25.** Verfahren zum Berechnen einer Elastizität nach irgendeinem der Ansprüche 17 bis 23, **dadurch gekennzeichnet, dass** es einen Schritt zum automatischen Erkennen des untersuchten Mediums durch das Berechnen von Gewebeparametern, wie zum Beispiel dem Ultraschall-Rückstreuungskoeffizienten, umfasst.

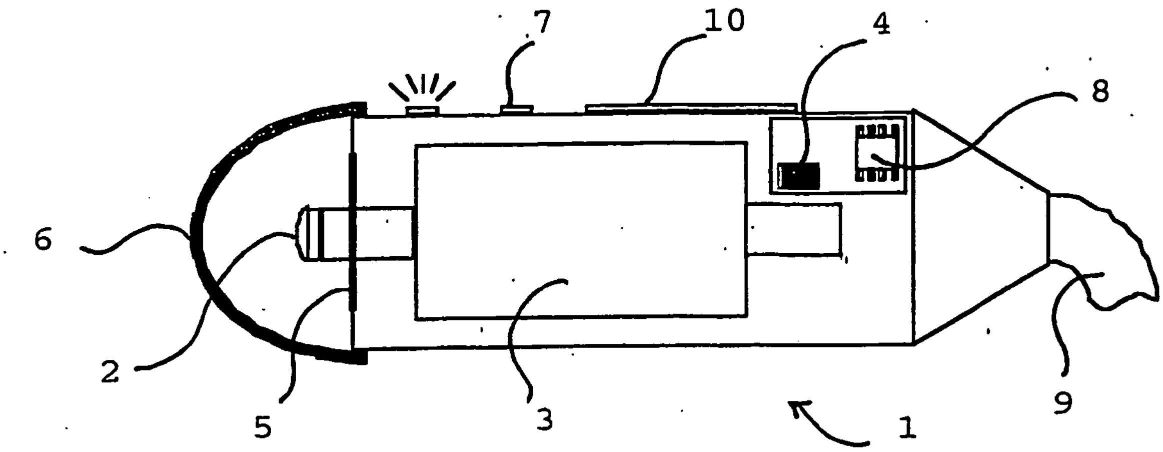


Figure 1

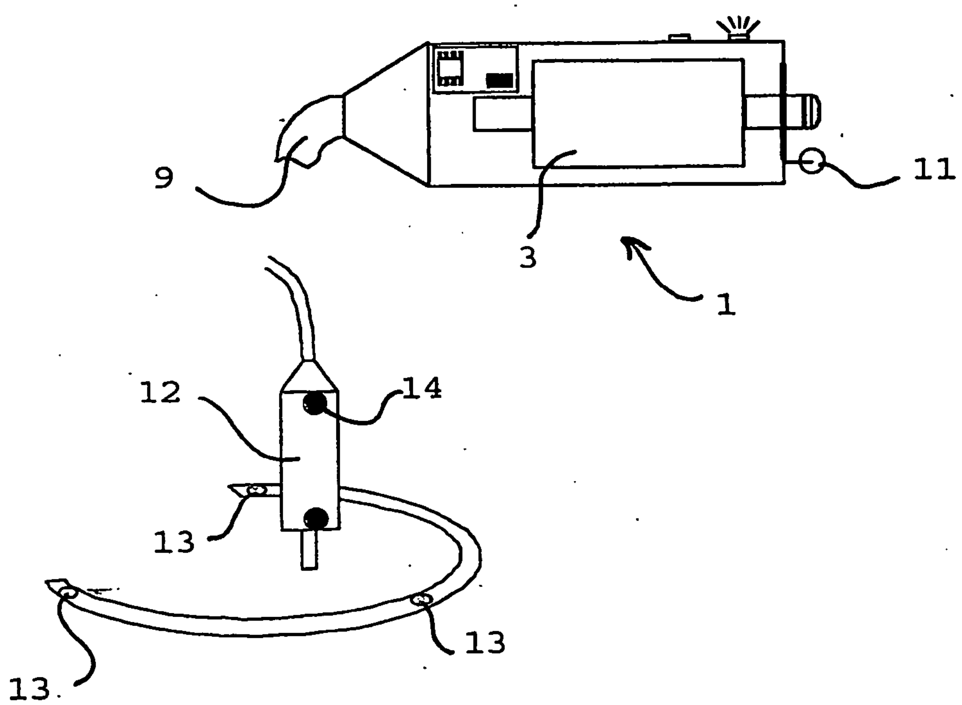


Figure 2

Figure 3

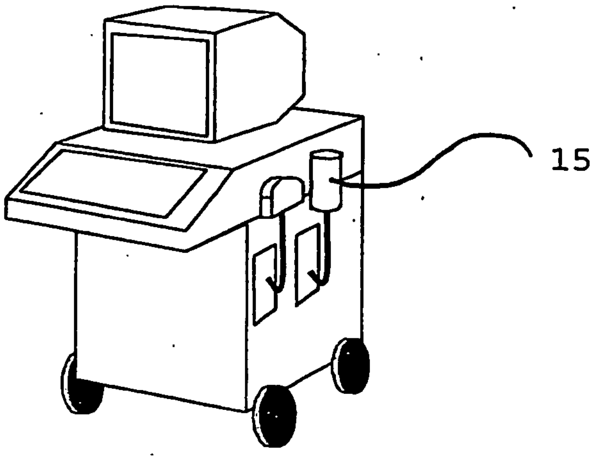


Figure 4

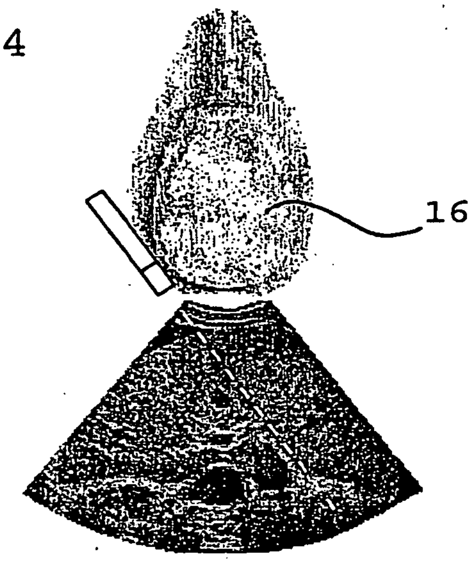


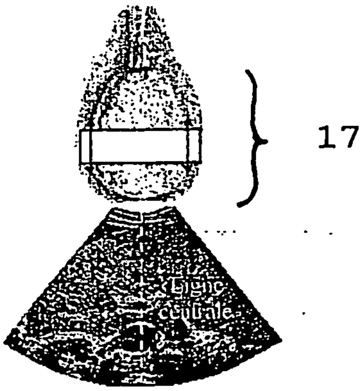


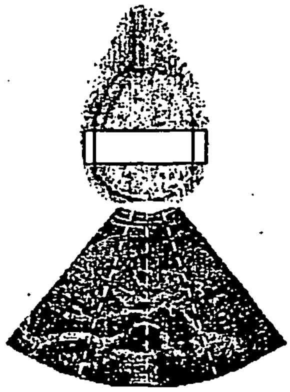

Figure 5a

Figure 5b

3/3

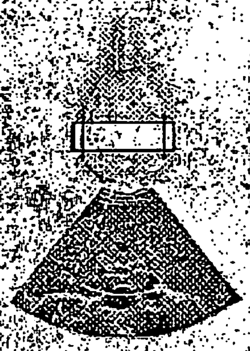

Figure 5c

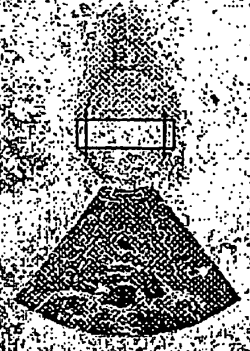

Figure 5d

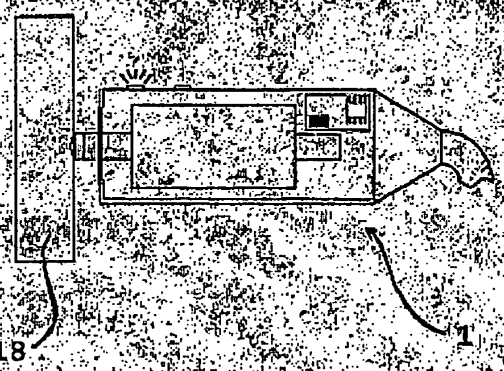


Figure 6

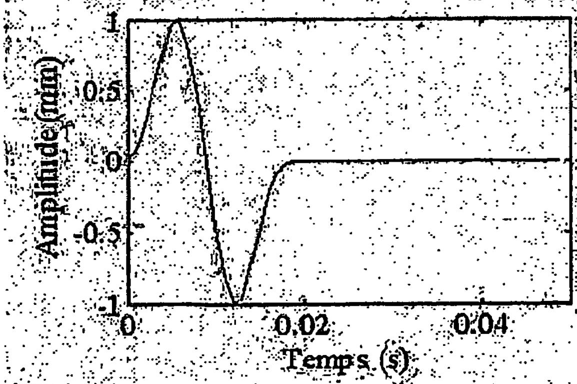


Figure 7a

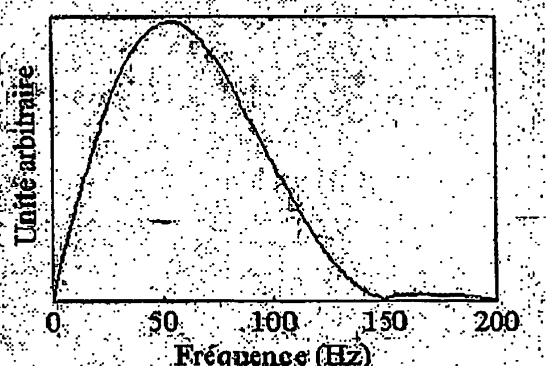


Figure 7b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5882302 A
- US 6277074 B
- US 5099848 A
- WO 0055616 A
- FR 9903157


### Littérature non-brevet citée dans la description


- **Shattuck.** a parrallel processing technique for high speed ultrasound imaging with linear phased arrays. *J. Acoust. Soc. Am.,* 1984, vol. 75 (4), 1273-1282